# FASCICULE DE BREVET EUROPEEN

(11) **EP 0 679 622 B1**
(45) Date de publication et mention de la délivrance du brevet: **28.10.1998**
(21) Numéro de dépôt: 95400903.1
(22) Date de dépôt: 24.04.1995
(51) Int. Cl.: C07C 5/373, B01J 23/96

(54) **Procédé catalytique de déshydroisomérisation de n-paraffines**
Katalytisches Verfahren zur Dehydroisomerisierung von n-Paraffinen
Catalytic process for the dehydroisomerisation of n-paraffins

(30) Priorité: 28.04.1994 FR 9405294
(43) Date de publication de la demande: 02.11.1995
(73) Titulaire: INSTITUT FRANCAIS DU PETROLE, 92502 Rueil-Malmaison (FR)
(72) Inventeur: Didillon, Blaise, F-92500 Rueil Malmaison (FR); Travers, Christine, F-92500 Rueil Malmaison (FR); Burzynski, Jean-Pierre, F-69110 Sainte-Foy-Les-Lyon (FR)

(56) Documents cités:
- GB-A- 2 246 524

## Description

La présente invention concerne l'utilisation d'un catalyseur particulier en déshydroisomérisation d'au moins une n-paraffine C₄-C₅, c'est-à-dire de n-butane et/ou de n-pentane, de préférence de n-butane, en une seule étape.

L'isobutène est un composé de première importance pour l'industrie pétrochimique. Un grand nombre de procédés de fabrication utilise l'isobutène comme matière première. On peut citer par exemple la synthèse de méthyltertiobutyl éther servant comme additif lors de la formulation des essences, la synthèse de méthacrylonitrile ou la synthèse de polymères.

Aujourd'hui la principale voie commerciale d'obtention de l'isobutène est un procédé en deux étapes à partir du n-butane. Le n-butane peut par exemple être isomérisé dans une première étape en isobutane (voir par exemple la demande de brevet français 2.695.635), l'isobutane étant déshydrogéné dans une seconde étape en isobutène (voir par exemple la demande de brevet européen EP-A-0.559.509). Une autre voie de production de l'isobutène à partir du n-butane est de déshydrogéner dans une première étape le n-butane en n-butène et d'isomériser le n-butène pour conduire à l'isobutène. Les catalyseurs, les conditions opératoires, les techniques de séparation utilisés pour ces différentes étapes sont généralement différents, ce qui impose de réaliser les deux étapes dans deux réacteurs séparés induisant par là même un coût élevé de production de l'isobutène à partir du n-butane.

La transformation directe du n-butane en isobutène présente donc un avantage certain par rapport aux procédés de préparation de l'isobutène en deux étapes. Les problèmes généralement rencontrés lors de ladite transformation sont d'une part le faible rendement obtenu et d'autre part la production parallèle de composés d'hydrogénolyse et/ou de craquage ainsi que celle de produits aromatiques. Enfin, la perte d'activité due au recouvrement de la surface active par des dépôts hydrocarbonés entraîne la nécessité de régénérer fréquemment le catalyseur.

Un certain nombre de catalyseurs a déjà été mentionné pour réaliser la transformation directe de n-butane en isobutène. Ces catalyseurs comprennent généralement un matériau zéolithique tel que par exemple les borosilicates (US-A-4.433.190, US-A-4.550.091), les aluminosolicates de type ZSM (EP-A-0.042.252), les zincoaluminates (US-A-4.962.266), ledit matériau étant utilisé seul ou en présence d'au moins un métal du groupe VIII (US-A-4.962.266) ou d'au moins un métal du groupe III (EP-A-0.042.252).

D'autres catalyseurs ont été aussi proposés, parmi lesquels les oxydes de chrome, de zirconium, de niobium ou de tantale (EP-A-0.192.059) ainsi que les catalyseurs comprenant un support tel que l'alumine, au moins un métal du groupe VIII tel que le platine, du silicium et éventuellement de l'étain et ou de l'indium (GB-A-2.246.524).

La présente invention concerne l'utilisation en déshydroisomérisation d'au moins une n-paraffine C₄-C₅, de préférence de n-butane, d'un catalyseur comprenant un support à base d'oxyde réfractaire telle que l'alumine, au moins un métal noble du groupe VIII, de préférence le platine ou le palladium, de manière encore plus préférée le platine, au moins un élément du groupe IVb tel que le titane ou le zirconium, de préférence le titane, au moins un élément du groupe formé par le germanium l'étain le plomb, le rhénium, le tungstène et l'indium, de préférence l'étain et au moins un halogène, de préférence le chlore. La présente invention concerne aussi la régénération dudit catalyseur.

L'utilisation selon l'invention concerne donc la déshydroisomérisation d'au moins une n-paraffine C₄-C₅, c'est-à-dire la transformation de ladite n-paraffine en une oléfine ramifiée.

La présente invention concerne de préférence l'utilisation en déshydroisomérisation d'au moins une n-paraffine C₄-C₅, de préférence de n-butane, d'un catalyseur comprenant un support à base d'oxyde réfractaire telle que l'alumine, au moins un métal noble du groupe VIII, de préférence le platine ou le palladium, de manière encore plus préférée le platine, au moins un élément du groupe IVb, titane ou zirconium, au moins un métal du groupe formé par le germanium, l'étain, le plomb, le rhénium, le tungstène et l'indium, de préférence l'étain, et au moins un halogène tel que par exemple le chlore. La présente invention concerne aussi la régénération dudit catalyseur.

La présente invention concerne aussi la régénération dudit catalyseur suite à sa désactivation après une longue période d'utilisation.

La surface spécifique du support à base d'oxyde réfractaire, de préférence à base d'alumine, est généralement avantageusement comprise entre 10 et 500 m²/g, de préférence entre 50 et 450 m²/g, tandis que son volume poreux est généralement compris entre 0,4 et 0,8 cm³/g.

La teneur dudit catalyseur utilisé selon l'invention en métal noble du groupe VIII, de préférence en platine ou en palladium, de manière préférée en platine, est comprise entre 0,1 et 5 % poids, de préférence entre 0,2 et 0,7 % poids. La teneur dudit catalyseur en élément du groupe IVB est généralement comprise entre 0,005 % et 0,2 % poids. Le catalyseur utilisé selon l'invention comprend au moins un métal du groupe formé par le germanium, l'étain, le plomb, le rhénium, le tungstène et l'indium, de préférence l'étain, la teneur dudit catalyseur en ledit métal est généralement comprise entre 0,1 et 5 % poids. Le catalyseur utilisé selon l'invention comprend au moins un halogène, de préférence le chlore, la teneur dudit catalyseur en ledit halogène est généralement comprise entre 0,001 et 6 %.

La préparation du catalyseur, utilisé selon l'invention, se fait selon des techniques bien connues de l'homme du métier. Néanmoins quelques précisions sont données ci-après.

Dans le cas de l'utilisation selon l'invention d'un catalyseur préféré comprenant un support à base d'alumine, au moins un métal noble du groupe VIII, de préférence le platine, et au moins un élément du groupe IVB, de préférence le titane, au moins un élément du groupe formé par le germanium, l'étain, le plomb, le rhénium, le tungstène et l'indium, de préférence l'étain, et au moins un halogène, de préférence le chlore, on peut utiliser pour la préparation dudit catalyseur des alumines commerciales, de préférence activées, choisies de manière préférée dans le groupe des alumines éta et gamma, et ayant une faible teneur en alcalins, par exemple contenant moins de 0,1 % poids de sodium. Ledit catalyseur est généralement préparé en ajoutant, dans une étape de préparation d'un support modifié (c'est-à-dire d'un support à base d'alumine comprenant au moins un métal du groupe IVB, de préférence le titane), de 0,05 à 1 % et de préférence de 0,085 à 0,5 % d'oxyde, dudit élément du groupe IVB, de préférence de bioxyde de titane, sur le support. Tout procédé permettant ledit ajout convient. On peut, par exemple, dissoudre un composé dudit élément du groupe IVB, de préférence de titane, dans la solution contenant le composé d'aluminium et ajuster les conditions de précipitation de l'alumine pour que l'hydroxyde dudit élément du groupe IVB, de préférence de titane, coprécipite. On peut également ajouter à l'alumine hydratée sous forme de gel (α-trihydrate, β-trihydrate ou α-monohydrate d'aluminium) au moins un composé dudit élément du groupe IVB, de préférence de titane, par exemple choisi, dans le cas préféré où ledit élément du groupe IVB est le titane, dans le groupe formé par le bioxyde de titane sous les formes rutile et anatase, les sous-oxydes TiO et Ti₂O₃, les acides titaniques, les titanates alcalins, alcalino-terreux et d'ammonium et les sels solubles et insolubles, organiques et inorganiques du titane. On peut également partir d'un support à base d'alumine mis en forme et l'imprégner avec une solution d'un sel organique ou inorganique dudit élément du groupe IVB, de préférence de titane ; d'une façon générale, l'addition dudit élément du groupe IVB, de préférence de titane, peut être conduite avant la mise en forme, au cours de la mise en forme ou après mise en forme du support de catalyseur.

Un premier procédé préféré de préparation dudit support modifié, dans le cas où ledit élément du groupe IVB est le titane, consiste à ajouter à une solution organique (par exemple alcoolique) d'au moins un composé organique d'aluminium (par exemple un alcoxyaluminium tel que l'isopropylate d'aluminium), au moins un composé organique du titane, par exemple le tétraéthoxytitane, puis à hydrolyser la solution ainsi obtenue. On peut également dans ledit cas ajouter le titane sous forme d'un composé inorganique facilement hydrolysable tel que le tétrachlorure de titane TiCl₄.

Un second procédé préféré de préparation dudit support modifié, dans le cas où ledit élément du groupe IVB est le titane, consiste à ajouter en quantités contrôlées un composé organique à base de titane, par exemple un alcoxytitane tel que le tétraéthyltitane et/ou un composé inorganique du titane (par exemple le trichlorure de titane) au cours de la synthèse Ziegler du polyalcoxyaluminium, par réaction d'un alkylaluminium, (par exemple le triéthylaluminium), de l'éthylène, et d'au moins un composé précité du titane. Par polymérisation puis oxydation subséquente, on prépare le polyalcoxyaluminium précité, dont l'hydrolyse conduira aux polyols et à l'alumine hydratée contenant du titane.

Il a été constaté expérimentalement que les deux procédés préférés d'imprégnation du titane précédemment décrits, conduisaient à une dispersion particulièrement élevée des ions titane dans la matrice d'alumine, obtenue après hydrolyse de l'alcoxyaluminium ou du polyalcoxyaluminium. Lesdits procédés préférés d'imprégnation du titane précédemment décrits, permettent, par exemple, lorsque le support se présente sous une forme telle qu'une forme de bille ou d'extrudé par exemple, d'obtenir une teneur en TiO₂ constante d'une bille à l'autre ou d'un extrudé à l'autre ; si la concentration moyenne désirée est C %, la concentration, C, d'une bille à l'autre ou d'un extrudé à l'autre reste, avec les méthodes préférées de l'invention généralement comprise entre C ± 5 % en poids de cette concentration et même comprise entre C ± 3 % en poids. Des résultats encore améliorés ont été obtenus en utilisant des supports modifiés de catalyseur contenant plus particulièrement de 0,06 à 0,15 % TiO₂, la teneur en titane sur le support étant mesurée par fluorescence X.

Le support ainsi obtenu est ensuite séché à une température comprise entre 100 et 130°C et éventuellement calciné sous air à une température comprise entre 400 et 800°C, de préférence entre 450 et 750°C, pour une durée comprise entre 1 et 5 heures. Il peut ensuite être avantageusement traité sous vapeur d'eau à une température comprise entre 120 et 700°C, de manière préférée entre 300 et 700°C, sous une pression partielle de vapeur d'eau supérieure à 50 kPa et de préférence comprise entre 60 et 100 kPa pour une durée comprise entre 0,5 et 120 heures, de préférence entre 1 et 100 heures.

Le métal noble du groupe VIII, de préférence le platine, est introduit selon toute technique connue de l'homme du métier. Ledit métal noble est par exemple introduit par des techniques d'imprégnation de solutions aqueuses ou organiques d'un précurseur dudit métal noble, ledit précurseur pouvant être un composé minéral tel que par exemple dans le cas où ledit métal noble est le platine, l'acide hexachloroplatinique, le platine tetramine dihydroxyde, le chlorure de platine tetramine, ou un composé organométallique tel que par exemple le platine bis p allyl, le platine bis acétylacétonate. Après introduction dudit métal noble, le catalyseur peut éventuellement être séché, calciné à une température comprise entre 300 et 700°C, de préférence entre 350 et 550°C, et/ou réduit à une température comprise entre 300 et 700°C, de préférence entre 350 et 550°C.

L'élément du groupe formé par le germanium, l'étain, le plomb, le rhénium, le tungstène et l'indium, de préférence l'étain, ledit élément peut par exemple être incorporé par imprégnation de ce support à l'aide d'une solution adéquate, aqueuse ou organique, renfermant un sel ou un composé dudit élément tel qu'un chlorure, un acétate, un tartrate ou un alkoyl.

L'halogène est introduit par exemple par imprégnation sur le support d'une solution aqueuse d'acide contenant un halogène telle une solution aqueuse d'acide chlorhydrique.

La réaction de déshydroisomérisation d'au moins une n-paraffine C₄-C₅, de préférence de n-butane, est réalisée, dans une zone de déshydroisomérisation, en passant une charge comprenant au moins une n-paraffine C₄-C₅, de préférence du n-butane, sur le catalyseur à haute température dans une zone de déshydroisomérisation. Avant réaction, le catalyseur peut éventuellement être réduit. Généralement la réaction de déshydroisomérisation est conduite à une température comprise entre 250 et 600°C, de préférence entre 400 et 600°C, à une pression totale comprise entre 10 et 5000 kPa, de préférence entre 30 et 200 kPa, avec un rapport molaire hydrogène/hydrocarbure(s) compris entre 0 et 10 et avec un débit volumique, exprimé en volume gazeux de charge par heure et par volume de catalyseur, compris entre 30000 et 75 h⁻¹, de préférence entre 15000 et 200 h⁻¹.

Le catalyseur utilisé selon l'invention peut être régénéré après une période d'utilisation. La régénération du catalyseur est réalisée par combustion contrôlée des espèces hydrocarbonées présentes sur le catalyseur. Cette combustion est réalisée dans les conditions connues de l'homme du métier, généralement en chauffant progressivement le catalyseur en présence d'un gaz contenant de l'oxygène à une température comprise entre 350 et 500°C. La régénération inclut à l'issue de l'étape de combustion du coke une étape d'oxyhalogénation, de préférence d'oxychloration, du catalyseur, qui consiste à introduire à la fin de la combustion au moins un dérivé halogéné, c'est-à-dire au moins un halogène et/ou un composé halogéné, de préférence au moins du chlore et/ou un composé chloré. La quantité d'halogène utilisée correspond à une valeur comprise entre 0,1 et 2 g d'halogène pour 100 g de catalyseur. Pour une telle réaction d'oxyhalogénation, la température est généralement comprise entre 350 et 650°C et la pression est généralement comprise entre 101 et 1500 kPa.

Les exemples qui suivent permettent d'illustrer l'invention.

### Exemple 1 (comparatif) : Préparation du catalyseur A

Un support d'alumine gamma commercial, de surface 200 m²/g, et de volume poreux 0,6 cm³/g, est imprégné de 0,1 % de titane à partir d'oxalate de titane décahydraté en solution aqueuse, puis séché à 100°C pendant 2 heures et calciné à 600°C pendant 2 heures. Le support ainsi obtenu est ensuite soumis à un traitement à la vapeur d'eau à 560°C pendant 20 heures avec une pression partielle de vapeur d'eau égale 80 kPa. On dépose ensuite sur ce support 0,6 % de Pt selon les techniques de l'art antérieur :

A 100 g de support on ajoute 500 cm³ d'une solution aqueuse d'acide chlorhydrique. On laisse en contact trois heures, on essore, on sèche 1 heure à 120°C. Sur le produit séché contenant du chlore, on procède alors à l'imprégnation du platine en ajoutant au solide 150 cm³ d'une solution d'acide hexachloroplatine. La concentration en platine de cette solution est égale à 4,05 g par litre. On laisse en contact 6 heures, on sèche 1 heure à 120°C puis on calcine 2 heures à 530°C.

Le produit ainsi obtenu, catalyseur A, comprend 0,6 % de platine, 0,1 % de titane et 1,2 % de chlore (% poids).

### Exemple 2. Préparation du catalyseur B selon l'invention.

Le support utilisé dans cet exemple est équivalent au support décrit dans l'exemple 1. On dépose alors le platine et l'étain selon les techniques de l'art antérieur.

A 100 g de support on ajoute 500 cm³ d'une solution aqueuse d'acide chlorhydrique. On laisse en contact 3 heures, on essore, on sèche 1 heure à 120°C. Sur le produit séché contenant du chlore, on procède alors à l'imprégnation du platine et de l'étain en ajoutant au solide 150 cm³ d'une solution d'acide hexachloroplatine et de chlorure stannique. La concentration en platine de cette solution est égale à 4,05 g par litre et la concentration en étain est de 3,04 g par litre. On laisse en contact 6 heures, on sèche 1 heure à 120°C puis on calcine 2 heures à 530°C.

Le produit ainsi obtenu, catalyseur B, comprend 0,6 % de platine, 0,1 % de titane, 0,3 % d'étain et 1,2 % de chlore (% poids).

### Exemple 3 (comparatif) : Préparation du catalyseur C

Le catalyseur utilisé dans cet exemple diffère du catalyseur utilisé dans l'exemple 1 uniquement en ce que le support d'alumine gamma commercial n'est pas imprégné de titane. Toutes les étapes ultérieures de la préparation sont identiques à celles de l'exemple 1.

Le produit ainsi obtenu, catalyseur C, comprend 0,6 % de platine et 1,2 % de chlore (% poids).

### Exemple 4 (comparatif) : Préparation du catalyseur D

Un catalyseur est préparé à partir de 300 g de support alumine imprégné de titane selon ce qui a été décrit à l'exemple 1. Puis on procède à l'introduction du platine par ajout de 1500 cm3 d'une solution organique (toluène) de platine bis acétylacétonate contenant en tout 1.8 g de platine. On laisse 2 heures en contact, on filtre et on sèche 1 heure à 120° C puis on calcine pendant 4 heures à 400° C. Le produit ainsi obtenu, catalyseur D, comprend 0,6 % de platine et 0,1 % de titane.

### Exemple 5. Comparaison des performances des différents catalyseurs en déshydroisomérisation du n-butane.

1 g du catalyseur à tester est placé dans un réacteur en quartz d'un diamètre interne de 20 mm fonctionnant en flux ascendant à la pression atmosphérique. Le catalyseur est d'abord réduit à 350°C sous un débit d'hydrogène de 1 litre par heure pendant deux heures. Après cette réduction la température est amenée à 530°C. On injecte un mélange gazeux contenant du n-butane, de l'hydrogène et de l'azote. Le rapport molaire hydrogène/n-butane est de 1. Le rapport n-butane/hydrogène est de 0,5. Le débit de gaz exprimé en volume de n-butane par heure et par litre de catalyseur est de 2750 h⁻¹. La température est alors stabilisée à 550°C. Les résultats d'analyse de prélèvements gazeux réalisés après 4 heures de fonctionnement sont rapportés dans le tableau 1.

**TABLEAU 1**

| Catalyseur | Conversion (%) | Rendement (% poids) | |
|---|---|---|---|
| | | i-butène | butènes |
| A | 52 | 21 | 59 |
| B | 90 | 28 | 71 |
| C | 37 | 15 | 52 |
| D | 27 | 3 | 27 |

### Exemple 6. Régénération des catalyseurs A et B usés

Après utilisation du catalyseur A ou B pendant 12 heures dans les conditions de l'exemple 5, le catalyseur A ou B est régénéré. Dans cette procédure de régénération le catalyseur A ou B est porté à 200°C sous azote, puis traité sous air dilué à une température comprise entre 200 et 500°C pour brûler les composés hydrocarbonés présents sur le catalyseur. Le catalyseur A ou B est ensuite soumis à un traitement à la vapeur d'eau à 560°C pendant 20 heures avec une pression partielle de vapeur d'eau égale à 0,8 bar.

Après régénération, les performances des catalyseurs A et B régénérés sont évaluées dans les conditions de l'exemple 5. Les performances de tels systèmes régénérés sont rapportées dans le tableau 2.

**TABLEAU 2**

| Catalyseur | Conversion (%) | Rendement (% poids) | |
|---|---|---|---|
| | | i-butène | butènes |
| A | 52 | 21 | 59 |
| A - régénéré | 30 | 19 | 53 |
| B | 90 | 28 | 71 |
| B - régénéré | 87 | 29 | 73 |

## Revendications

1. Utilisation en déshydroisomérisation d'au moins une paraffine C₄-C₅ d'un catalyseur comprenant un support à base d'oxyde réfractaire, au moins un métal noble du groupe VIII, au moins un élément du groupe IVb, choisi parmi le titane et le zirconium, au moins un métal du groupe formé par le germanium, l'étain, le plomb, le rhénium, le tungstène et l'indium et au moins un halogène, ladite réaction étant réalisée dans une zone de déshydroisomérisation, en passant une charge d'hydrocarbures comprenant au moins une paraffine C₄-C₅ sur ledit catalyseur.

2. Utilisation selon la revendication 1, à une température comprise entre 250 et 600°C, à une pression totale comprise entre 10 et 5000 kPa, avec un rapport molaire hydrogène/hydrocarbure(s) compris entre et 0 et 10 et avec un débit volumique, exprimé en volume gazeux de charge par heure et par volume de catalyseur, compris entre 30000 et 75 h⁻¹.

3. Utilisation selon l'une des revendications 1 ou 2 telle que ledit métal noble du groupe VIII est le platine ou le palladium.

4. Utilisation selon l'une des revendications 1 à 3 telle que ledit métal noble du groupe VIII est le platine.

5. Utilisation selon l'une des revendications 1 à 4 telle que ledit halogène est le chlore.

6. Utilisation selon l'une des revendications 1 à 5 telle que ledit métal est l'étain.

7. Utilisation selon l'une des revendications 1 à 6 telle que ladite n-paraffine est le n-butane.

8. Utilisation selon l'une des revendications 1 à 7 telle que le support est l'alumine.

9. Régénération d'un catalyseur comprenant un support à base d'oxyde réfractaire, au moins un métal noble du groupe VIII, au moins un élément du groupe IVb choisi parmi le titane et le zirconium, au moins un métal du groupe formé par le germanium, l'étain, le plomb, le rhénium, le tungstène et l'indium, et au moins un halogène, ledit catalyseur ayant été utilisé en déshydroisomérisation d'au moins une paraffine C₄-C₅ selon l'une des revendications 1 à 8, réalisée en chauffant progressivement ledit catalyseur en présence d'un gaz contenant de l'oxygène à une température comprise entre 350 et 500° C.

## Claims

1. The use, for the dehydroisomerisation of at least one C₄-C₅ paraffin, of a catalyst containing a refractory oxide based support, at least one precious metal from group VIII and at least one element from group IVB, selected from titanium and zirconium, at least one metal selected from the group formed by germanium, tin, lead, rhenium, tungsten and indium and at least one halogen, said reaction being carried out in a dehydroisomerisation zone by passing a hydrocarbon feed containing at least one C₄-C₅ paraffin over said catalyst.

2. Use according to claim 1, at a temperature of between 250°C and 600°C, at a total pressure of between 10 and 5000 kPa, with a hydrogen/hydrocarbon molar ratio of between 0 and 10 and with a volume flow rate, expressed as the volume of gaseous feed per hour per volume of catalyst, of between 30000 and 75 h-1.

3. Use according to claim 1 or claim 2, wherein said precious metal from group VIII is platinum or palladium.

4. Use according to any one of claims 1 to 3, wherein said precious metal from group VIII is platinum.

5. Use according to claim 1 to 4, wherein said halogen is chlorine.

6. Use according to claim 1 to 5, wherein said metal is tin.

7. Use according to any one of claims 1 to 6, wherein said n-paraffin is n-butane.

8. Use according to any one of claims 1 to 7, wherein the support is alumina.

9. The regeneration of a catalyst comprising a refractory oxide based support and at least one precious metal from group VIII, at least one element from group IVB selected from titanium and zirconium, at least a metal selected from the groups formed by germanium, tin, lead, rhenium, tungsten and indium, and at least one halogen, said catalyst having been used for the dehydroisomerisation of at least one C₄-C₅ paraffin in accordance with any one of claims 1 to 10, effected by slowly heating said catalyst in the presence of a gas containing oxygen to a temperature of between 350°C and 500°C.

## Patentansprüche

1. Verwendung eines Katalysators, der einen Träger auf Basis eines schwerschmelzbaren (feuerfesten) Oxids, mindestens ein Edelmetall aus der Gruppe VIII, mindestens ein Element der Gruppe IVb, ausgewählt aus Titan und Zirkonium, mindestens ein Metall aus der Gruppe Germanium, Zinn, Blei, Rhenium, Wolfram und Indium und mindestens ein Halogen umfaßt, bei der Dehydroisomerisierung mindestens eines C₄-C₅-Paraffins, wobei die genannte Reaktion in einer Dehydroisomerisierungszone durchgeführt wird, in der man eine Kohlenwasserstoff-Beschickung, die mindestens ein C₄-C₅-Paraffin enthält, über den genannten Katalysator leitet.

2. Verwendung nach Anspruch 1 bei einer Temperatur zwischen 250 und 600°C, bei einem Gesamtdruck zwischen 10 und 5000 kPa mit einem Wasserstoff/Kohlenwasserstoff(en)-Molverhältnis zwischen 0 und 10 und mit einem Volumendurchsatz, ausgedrückt in Gasvolumen der Beschickung pro Stunde und pro Volumen Katalysator, zwischen 30 000 und 75 h⁻¹.

3. Verwendung nach einem der Ansprüche 1 oder 2, bei der das genannte Edelmetall der Gruppe VIII Platin oder Palladium ist.

4. Verwendung nach einem der Ansprüche 1 bis 3, bei der das genannte Edelmetall der Gruppe VIII Platin ist.

5. Verwendung nach einem der Ansprüche 1 bis 4, bei der das genannte Halogen Chlor ist.

6. Verwendung nach einem der Ansprüche 1 bis 5, bei der das genannte Metall Zinn ist.

7. Verwendung nach einem der Ansprüche 1 bis 6, bei der das genannte n-Paraffin n-Butan ist.

8. Verwendung nach einem der Ansprüche 1 bis 7, bei der der Träger Aluminiumoxid ist.

9. Regenerierung eines Katalysators, der einen Träger auf Basis eines schwerschmelzbaren (feuerfesten) Oxids, mindestens ein Edelmetall der Gruppe VIII, mindestens ein Element der Gruppe IVb, ausgewählt aus Titan und Zirkonium, mindestens ein Metall der Gruppe Germanium, Zinn, Blei, Rhenium, Wolfram und Indium und mindestens ein Halogen umfaßt, wobei der genannte Katalysator bei der Dehydroisomerisierung mindestens eines C₄-C₅-Paraffins nach einem der Ansprüche 1 bis 8 verwendet worden ist, die durchgeführt wird, indem man den genannten Katalysator in Gegenwart eines Sauerstoff enthaltenden Gases fortschreitend auf eine Temperatur zwischen 350 und 500°C erwärmt.
